# EUROPEAN PATENT APPLICATION

(11) **EP 3 654 338 A1**
(43) Date of publication of application: **20.05.2020**
(21) Application number: 18206488.1
(22) Date of filing: 15.11.2018
(51) Int. Cl.: G16H 10/60, G16H 10/40, G06Q 50/00

(54) **METHOD AND DEVICES FOR EXCHANGING HEALTH DATA**

(71) Applicant: F. Hoffmann-La Roche AG, 4070 Basel (CH); Roche Diagnostics GmbH, 68305 Mannheim (DE)
(72) Inventor: REIMERS, Jacob, 6343 Rotkreuz (CH)
(74) Representative: Altmann Stößel Dick Patentanwälte PartG mbB

(57) **Abstract**

A method (110) for exchanging health data of a patient in a medical system (112) is disclosed. The medical system (112) is accessible by one or more users including at least the patient. The method (110) comprises the following steps:
- at least one authentication step (114), wherein identification data is generated by a mobile application (116) of a mobile device (118) of the patient, wherein the identification data is transmitted to an authentication unit (120) by the mobile application (116);
- at least one authorization step (122), wherein access rights of the users of the medical system (112) to access a data server (124) are assigned by an authorization unit (126);
- at least one association step (128), wherein, upon successful authentication of the patient and successful authorization of the patient, a biological sample (130) of the patient is associated with the patient, wherein at least one sample identity information is provided to the data server (124) by the mobile application (116);
- at least one health data receiving step (132), wherein, upon successful authorization by the authorization unit (126), at least one analytical result of the biological sample (130) is received by the data server (124);
- at least one health data access step (134), wherein, upon successful authentication of the patient and successful authorization of the patient, the data server (124) permits the patient to access to the analytical result via the mobile application (116).

## Description

### TECHNICAL FIELD

The present disclosure relates to a method for exchanging health data. The present invention furthermore relates to a computer program, a mobile device, a server system and a medical system for exchanging health data.

### BACKGROUND

Health data such as laboratory results need to be exchanged between the patient and third parties such as laboratories, physicians or healthcare systems. Ownership of the health data is by the respective patient himself such that access to his health data should be under control of the patient. In particular, access to health data such as who is allowed to access health data should be dependent on whether the patient has granted consent.

The following scenario is known for exchange of health data: A patient provides a sample to a physician who sends the sample for testing to a laboratory. The laboratory result is send back to the physician and the patient receives the laboratory result from the physician. The data exchange conventionally is paper based or based on Portable Document Format (PDF). Paper based exchange of health data may be unsecure, costly and time consuming. Furthermore, despite progress in electronic data transfer, specifically, for exchange of health data several issues remain.

In particular, the patient gives all his health data to a third party without knowing how and if the data is protected and what is further done with his health data. Data security is still an open issue and has to be ensured, specifically, in order to prevent non-granted access e.g. by criminal elements. Furthermore, each of the individual parties involved in health data exchange has its own IT system which is non-coordinated with the others. Thus, it is up to the third parties to enforce access rights. Each of the individual parties is responsible for maintaining security and exchange data with the others. Exchange of health data between the systems may be difficult since the systems do not agree on the identity of the owner of the health data. No shared exchange format exists such that generation, distribution and processing of health data may be non-efficient, non-uniform and non-standardized. In addition, it may be difficult to integrate control by the patient into decision making processes.

Processes and systems of managing health data are known using a centralized computer. US 7,698,154 B2 describes a system and process for providing a computerized medical and biographical records database and diagnostic information. A medical records database and diagnostic program is stored on a central computer that is accessible to individuals using remotely situated computers connected to a computer network. Individual patient medical and biographical records are owned by individual patients who can enter information in their record as well as grant or deny authorization to others, such as health care professionals, insurance providers and other entities, to review part or all of their record. The diagnostic program provides a series of diagnostic questions to an individual who must respond either "yes" or "no" to each question. Each potential response is weighted relative to its importance to a particular disease diagnosis. Relative weights for all responses to diagnostic questions are summed to identify potential diagnoses connected to the answered questions. The diagnostic program provides the individual with a list of potential diagnoses as well as permitting the individual to save the information to his or her individual medical and biographical record.

However, using a centralized system wherein the database is maintained on a central computer may be susceptible for problems with respect to accessibility, for attacks and for failures based on local errors. Moreover, in these known methods and systems the patient has to upload the health data on its own which may be susceptible for errors.

### SUMMARY

This problem is addressed by a computer program, a mobile device, a server system and a medical system for exchanging health data with the features of the independent claims. Advantageous embodiments which might be realized in an isolated fashion or in any combination are listed in the dependent claims.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect of the present invention a method for exchanging health data of a patient in a medical system is disclosed. The medical system is accessible by one or more users including at least the patient.

The term "health data" as used herein is a broad term and is to be given its ordinary and customary meaning to a person of ordinary skill in the art and is not to be limited to a special or customized meaning. The term specifically may refer, without limitation, to information relating to one or more of health condition or a medical information such as at least one medical record, at least one laboratory result, at least one diagnosis, or other health related data. The health data may be or may comprises health information being based on at least one analytical result of at least one analysis of at least one biological sample of the patient. The health data may be electronic health data.

The term "medical system for exchange of health data" as used herein is a broad term and is to be given its ordinary and customary meaning to a person of ordinary skill in the art and is not to be limited to a special or customized meaning. The term specifically may refer, without limitation, to a system configured to exchange health data between different users of the medical system. The medical system may comprise at least one server system and at least one client device such as a mobile device, as will be described in detail below. The term "exchange" as used herein is a broad term and is to be given its ordinary and customary meaning to a person of ordinary skill in the art and is not to be limited to a special or customized meaning. The term specifically may refer to sharing of health data between users of the medical system and/or providing access to health data between users of the medical system. The term "user" as used herein is a broad term and is to be given its ordinary and customary meaning to a person of ordinary skill in the art and is not to be limited to a special or customized meaning. The term specifically may refer, without limitation, to a person or machine using the medical system. The user may be one or more of the patient, a third party such as medical personnel, laboratory personnel, or one or more machines, for example, other systems such as a health care computer or a laboratory computer.

The method comprises the steps disclosed in the following. The steps may specifically be performed in the given order. Still, a different order is possible. The method may comprise additional steps which are not mentioned. It is further possible to perform one or more or all of the method steps repeatedly. Further, two or more of the method steps may be performed simultaneously or in a timely overlapping fashion.

The method comprises the following steps:
- at least one authentication step, wherein identification data is generated by a mobile application of a mobile device of the patient, wherein the identification data is transmitted to an authentication unit by the mobile application;
- at least one authorization step, wherein access rights of the users of the medical system to access a data server are assigned by an authorization unit;
- at least one association step, wherein, upon successful authentication of the patient and successful authorization of the patient, the biological sample of the patient is associated with the patient, wherein at least one sample identity information is provided to the data server by the mobile application;
- at least one health data receiving step, wherein, upon successful authorization by the authorization unit, at least one analytical result of the biological sample is received by the data server;
- at least one health data access step, wherein, upon successful authentication of the patient and successful authorization of the patient, the data server permits the patient to access to the analytical result via the mobile application.

The term "authentication step" as used herein is a broad term and is to be given its ordinary and customary meaning to a person of ordinary skill in the art and is not to be limited to a special or customized meaning. The term specifically may refer, without limitation, to verifying identity of the respective user and/or assigning identity to a user. As used herein, the term "authentication unit" is a broad term and is to be given its ordinary and customary meaning to a person of ordinary skill in the art and is not to be limited to a special or customized meaning. The term specifically may refer, without limitation, to an arbitrary unit or entity configured to generate and/or to provide trusted identity information to other units such as the authorization unit and the data server of the medical system. The authentication unit may be a server configured to provide trusted identity information. The authentication unit may act as identity provider. The authentication unit, the authorization unit and the data server may form a federation or distributed network, wherein the authorization unit and the data server trust the identity provider to define identities, i.e. to define who is who. In the authentication step the authentication unit may provide trusted identity information to the data server and the authorization unit. The term "trusted identify information" as used herein is a broad term and is to be given its ordinary and customary meaning to a person of ordinary skill in the art and is not to be limited to a special or customized meaning. The term specifically may refer, without limitation, to identity information proofed by the authentication unit. For example, the trusted identity information may be and/or may comprise at least one identity token. As used herein, the term "successful authentication" is a broad term and is to be given its ordinary and customary meaning to a person of ordinary skill in the art and is not to be limited to a special or customized meaning. The term specifically may refer, without limitation, to proof and/or verification of the identity by the authentication unit.

The term "mobile device" as used herein is a broad term and is to be given its ordinary and customary meaning to a person of ordinary skill in the art and is not to be limited to a special or customized meaning. The term specifically may refer, without limitation, to a mobile electronics device, more specifically to a mobile communication device such as a cell phone or smart phone. Additionally or alternatively, as will be outlined in further detail below, the mobile device may also refer to a tablet computer or another type of portable computer. The term "mobile application" as used herein is a broad term and is to be given its ordinary and customary meaning to a person of ordinary skill in the art and is not to be limited to a special or customized meaning. The term specifically may refer, without limitation, to an application, e.g. an app, including computer-executable instructions.

The mobile device may comprise at least one data transmission module adapted to transmit the identification data to the authentication unit. The identification data may be transmitted from mobile device to the authentication unit by use of a network such as the Internet, a local area network (LAN), a wide area network (WAN), or other type of network. For example, the transmission may use at least one communication protocol such as the transmission control protocol/Internet protocol (TCP/IP).

The term "the identification data" as used herein is a broad term and is to be given its ordinary and customary meaning to a person of ordinary skill in the art and is not to be limited to a special or customized meaning. The term specifically may refer, without limitation, to data identifying the respective user. The authentication step may be based on OpenID Connect standards. The identification data may be obtained using an OpenID Connect flow and/or is a phone number. For example, mobile application may request the patient to enter his or her phone number. The mobile application may be configured to transmit the identification to the authentication unit upon approval and/or consent by the patient, wherein, for example, the approval may be given by pressing at least one button of the mobile device or on a display device of the mobile device. The identification data may be confirmed by code sent by Short Message Service (SMS). For example, the authentication unit may be configured to transmit to the mobile device the at least one confirmation code. The confirmation code may be an arbitrary a piece of data such as a sequence of numbers and/or other characters configured for confirmation of the identity of the user. The mobile device may be configured to receive and/or display the confirmation code. The mobile application may be configured to allow the patient entering the confirmation code and to proceed with at least one further step of the method such as with the association step or the health data access step.

The term "authorization step" as used herein is a broad term and is to be given its ordinary and customary meaning to a person of ordinary skill in the art and is not to be limited to a special or customized meaning. The term specifically may refer, without limitation, to a step of assigning access rights to access the data server. The authorization unit may be configured for access control. As used herein, the term "access right" is a broad term and is to be given its ordinary and customary meaning to a person of ordinary skill in the art and is not to be limited to a special or customized meaning. The term specifically may refer, without limitation, to a selective permission or selective restriction of access to the data server. The authorization unit may determine which user can do what such as which user is allowed to access data and/or reading data and/or input data. The authorization unit may be configured to control access rights. Specifically, the authorization unit may determine, specifically decide, which data the user should be able to access and/or to which extend user should be able to access the data such as for reading, writing and/or for further actions. The term "authorization unit" as used herein is a broad term and is to be given its ordinary and customary meaning to a person of ordinary skill in the art and is not to be limited to a special or customized meaning. The term specifically may refer, without limitation, to a unit such as a server configured for authorization of a user. The authorization unit may act as an authorization provider. The authorization step may be based on an OAuth standard such an OAuth 2.0 standard. Use of an OAuth standard may allow applications to take actions on the data server on behalf of the respective user, such as may allow the mobile application of the mobile device to take actions on the data server on behalf of the patient. As used herein, the term "successful authorization" is a broad term and is to be given its ordinary and customary meaning to a person of ordinary skill in the art and is not to be limited to a special or customized meaning. The term specifically may refer, without limitation, to permission by the authorization unit.

The term "data server" as used herein is a broad term and is to be given its ordinary and customary meaning to a person of ordinary skill in the art and is not to be limited to a special or customized meaning. The term specifically may refer, without limitation, to a server unit or server system configured for storing health data. The data server may comprise at least one data storage device such as at least one memory for storing health data. The data server generally may comprise the at least one data storage device such as at least one volatile and/or at least one non-volatile data storage element. As an example, the data storage device, also referred to as a memory device or a memory element, may comprise one or more storage chips and/or other types of memory devices, wherein both volatile and non-volatile memory devices may be employed. The data server may comprise at least one database comprising the stored health data. As used herein, the term "to access the data server" used herein is a broad term and is to be given its ordinary and customary meaning to a person of ordinary skill in the art and is not to be limited to access to health data and/or to the data storage configured for storing the health data, specifically for one or more of: reading, writing, and/or for performing further actions.

The term "biological sample" as used herein is a broad term and is to be given its ordinary and customary meaning to a person of ordinary skill in the art and is not to be limited to a special or customized meaning. The term specifically may refer, without limitation, to an arbitrary biological specimen of the patient. The biological the sample may be one or more of a bodily fluid such as blood, urine, saliva, or other bodily fluids, a tissue sample, and other bodily samples such as hair specimen. The biological sample may be a drawn sample of the patient's body.

The term "association step" as used herein is a broad term and is to be given its ordinary and customary meaning to a person of ordinary skill in the art and is not to be limited to a special or customized meaning. The term specifically may refer, without limitation, to at least one process of linking the patient and the biological sample in the medical system. As further used herein, the term "association" is a broad term and is to be given its ordinary and customary meaning to a person of ordinary skill in the art and is not to be limited to a special or customized meaning. The term specifically may refer, without limitation, to linking, specifically uniquely linking, the patient with the biological sample. In the association step, the patient may register the biological sample through the mobile application. The authorization unit, upon successful authentication of the patient, may determine access rights of the patient and enables the patient to provide the sample identity information to the data server via the mobile application. The at least one sample identity information is provided to the data server by the mobile application. The association step may be based on OAuth 2.0 standards.

The method may further comprise identifying the biological sample with the mobile device by using a tag reader of the mobile device, specifically one or more of a camera or a near-field communication (NFC) reader. The mobile device may identify the biological sample by using a tag selected from the group consisting of a barcode and a rapid frequency identification device (RFID). As used therein, the term "identifying" generally refers to the process of recognizing one or more unique information assigned to the biological sample or any other information relating to the biological sample which characterizes or identifies the biological sample. As an example, identifying the biological sample may include reading a number attached or assigned to the biological sample which uniquely identifies the biological sample or characterizes the biological sample. Thus, the biological sample may have at least one unique identifier, designated as tag, wherein the process of identifying the biological sample implies reading the unique identifier. As further used herein, a "tag" generally refers to an information carrier which is capable of carrying information that uniquely identifies the biological sample to which the identifier is attached or assigned. The tag specifically may be an identifier which is contactless readable, such as an optical identifier and/or a contactless electronic identifier. As an example, the tag may comprise a one-dimensional, a two-dimensional or even a three-dimensional barcode. Additionally or alternatively, the at least one tag may comprise at least one rapid frequency identification device (RFID). Other examples are possible. The tag reader specifically may be configured for reading the tag. For reading the tag, the tag may be brought into a field of view of the tag reader. As an example, the tag reader may be configured as a camera, specifically a barcode reader, and/or as an NFC reader, specifically an RFID tag reader. The tag reader specifically may comprise at least one optical reader. As an example, the tag reader may comprise at least one barcode reader. The optical reader, as an example, may be a camera, specifically a scanner or may comprise a scanner, such as a two-dimensional scanner or a line-scanner.

As used herein, the term "near-field communication", generally refers to a wireless transfer of data over short distances of up to 10 cm, generally having a low data transfer rate, such as a data transfer rate of no more than 424 kBit/s. As an example, the near-field communication may follow a passive standard, i.e. a standard in which one of the communication partners is a passive component which only answers communication requests received from the other partner, such as the standard defined in ISO 14443 and/or ISO 15693. Preferably, the near-field communication may be a RFID communication, wherein, e.g., the NFC reader is the passive element of the RFID communication. Additionally or alternatively, other types of near-field communication may be used, such as near-field communications in which both partners of the communication are active partners, i.e. partners which may both send and receive communication requests. The NFC reader may comprise at least one communication component adapted to perform the near-field communication. As an example, the NFC reader may comprise at least one antenna. As an example, the NFC reader device may comprise at least one RFID antenna, such as at least one RFID coil. The NFC reader may further comprise additional components, such as one or more communication ICs or the like.

The method may further comprise transmitting the biological sample to an analyzer such as a laboratory for testing and/or analyzing the biological sample. The method further may comprise at least one analysis step, wherein the analytical result of the biological sample is generated by the analyzer. The analyzer may be or may comprise at least one arbitrary device configured for conducting at least one medical analysis and/or at least one medical procedure. The analyzer therefore may generally be an arbitrary device configured for performing at least one diagnostic purpose and/or at least one therapeutic purpose. The analyzer may be configured for performing at least one diagnostic purpose and, specifically may comprise at least one analyte sensor for performing at least one analysis. The analyzer may comprise an assembly of two or more components capable of interacting with each other, such as in order to perform one or more diagnostic and/or therapeutic purposes, such as in order to perform the medical analysis and/or the medical procedure. Specifically, the analyzer may be capable of performing at least one detection of the at least one analyte in the biological sample and/or in order to contribute to the at least one detection of the at least one analyte in the biological sample. The analyzer generally may be used for detecting at least one analyte in the biological sample of the patient. The analyzer may be or may comprise at least one of a sensor assembly, a sensor system, a sensor kit or a sensor device.

The term "generating the analytical result" as used herein is a broad term and is to be given its ordinary and customary meaning to a person of ordinary skill in the art and is not to be limited to a special or customized meaning. The term specifically may refer, without limitation, to determining the analytical result by analyzing the biological sample. For example, the analyzing may comprise at least one process of determining a presence and/or a quantity and/or a concentration of the at least one analyte. As further used herein, the term "analyte" is a component of a sample to be analyzed, e.g. molecules of various sizes, ions, proteins, metabolites and the like. Information gathered on an analyte may be used to evaluate the impact of the administration of drugs on the organism or on particular tissues or to make a diagnosis. Thus "analyte" is a general term for substances for which information about presence and/or concentration is intended. Examples of analytes are e.g. glucose, coagulation parameters, endogenic proteins (e.g. proteins released from the heart muscle), metabolites, nucleic acids and so on. The list of possible analysis results returned by the analyzer may comprise, without limitation, concentrations of the analyte in the sample, a digital (yes or no) result indicating the existence of the analyte in the sample (corresponding to a concentration above the detection level), optical parameters, DNA or RNA sequences, data obtained from mass spectrometry of proteins or metabolites and physical or chemical parameters of various types. Thus, the analysis may be or may comprise a qualitative detection, simply determining the presence of the at least one analyte or the absence of the at least one analyte, and/or may be or may comprise a quantitative detection, which determines the quantity and/or the concentration of the at least one analyte. As a result of the analysis, at least one information may be generated which characterizes an outcome of the analysis, such as at least one test result, e.g. a blood value, a blood test result, presence or absence of the analyte, or other health information. The term "analytical result" as used herein is a broad term and is to be given its ordinary and customary meaning to a person of ordinary skill in the art and is not to be limited to a special or customized meaning. The term specifically may refer, without limitation, to at least one test result determined by the analyzer.

The term "health data receiving step" as used herein is a broad term and is to be given its ordinary and customary meaning to a person of ordinary skill in the art and is not to be limited to a special or customized meaning. The term specifically may refer, without limitation, to at least one process of receiving the analytical result by the data server. The receiving of the analytical result may comprise storing the analytical result in the data server. Once the analytical result is ready, the analytical result can be provided to the patient. Upon successful authorization by the authorization unit, the at least one analytical result of the biological sample is received by the data server. The analyzer may automatically and/or directly upload the analytical result to the data server. This may remove patient action and, thus, may reduce user errors and making it more convenient for the patient. If the authorization unit successfully authorizes the analyzer to access the data server, the analyzer may provide the analytical result to the data server. The method further may comprise the analyzer providing authentication information to the authentication unit. As used herein, the term "authentication information" is a broad term and is to be given its ordinary and customary meaning to a person of ordinary skill in the art and is not to be limited to a special or customized meaning. The term specifically may refer, without limitation, to information enabling the authentication unit to authenticate the analyzer such as a password or other access key. The authorization unit, upon successful authentication of the analyzer by the authentication unit, may determine access rights of the analyzer and may enable the analyzer to provide the analytical result to the data server. The patient may provide analyzer access information to the authorization unit via the mobile application. As used herein, the term "analyzer access information" is a broad term and is to be given its ordinary and customary meaning to a person of ordinary skill in the art and is not to be limited to a special or customized meaning. The term specifically may refer, without limitation, to information with respect to consent and/or approval of access to the data server for the analyzer. The authorization unit may grant or deny permission to provide the analytical result to the data server based on said analyzer access information. The data server may allow or deny providing the analytical result based on permission from the authorization unit. The health data receiving step may be based on OAuth 2.0 standards and User Managed Access (UMA) protocol.

The term "health data access step" as used herein is a broad term and is to be given its ordinary and customary meaning to a person of ordinary skill in the art and is not to be limited to a special or customized meaning. The term specifically may refer, without limitation, to at least one process of receiving and/or retrieving health data. Upon successful authentication of the patient and successful authorization of the patient, the data server permits the patient to access to the analytical result via the mobile application. The data server may issue a notification to the patient that analytical results were uploaded and/or are available on the data server via the mobile application. The method further may comprise the mobile application displaying at least one item of health information to the patient by using at least one display device, the at least one item of health information being based on the analytical result.

The method may further comprise at least one controlling step. The controlling step may comprise the patient, via the mobile application, granting or denying access to the analytical result to other users of the medical system and/or the patient, via the mobile application, assigning access rights to the analytical result to other users of the medical system. For example, once the analytical result is available on the data server, the mobile application can access the data server to download the analytical result. The mobile application may allow the patient to grant or deny access to the analytical result to other users such as health care professionals. The patient may provide access information to the authorization unit via the mobile application. As used herein, the term "access information" is a broad term and is to be given its ordinary and customary meaning to a person of ordinary skill in the art and is not to be limited to a special or customized meaning. The term specifically may refer, without limitation, to information with respect to consent and/or approval of access to the data server for the other users of the medical system. The authorization unit may grant or deny permission based on said access information. The data server may allow or deny access to the analytical result based on permission from the authorization unit. One or both of the health data access step and the controlling step may be based on OAuth 2.0 standards and User Managed Access (UMA) protocol. The UMA protocol may define a method for the patient to introduce the health data to the authorization unit.The UMA protocol may define at least one policy for controlling access to the health data on the data server. The UMA protocol may request users of the medical system, e.g. the patient and other users of the medical system, to provide claims to fulfill the defined policies for accessing the data server, specifically the health data. The use of the UMA protocol may allow ensuring and increasing baseline security. In addition, the use of UMA protocol may enhance interoperability between entities of the medical system.

As outlined above, the method for exchange of health data may use a server system comprising authentication unit, authorization unit and data server which may form a federation or distributed network. For user access the method may use the HEART standards, specifically OpenID Connect to identify users, OAuth 2.0 to delegate access to trusted applications and UMA to control who can access the data. This may allow patient managed access. Patients can manage their consent online. Granting and retracting consent may be equally easy for patients. The use of the HEART standard may allow relying on existing identity and delegation standards, which are compliant with General Data Protection Regulation (GDPR). All parties agree on identities, so systems can exchange data with authorized people.

The proposed method may provide a decentralized solution where data is uploaded automatically and can be shared e.g. beyond the healthcare service, ex for parents managing child's disease. The fact that all information is not maintained on a single central computer may improve security and accessibility. Moreover, the health data can be stored and secured in a specialized location. Therefore, there is a lower risk of failures based on local errors and splitting results from person adds additional layer of protection. Demographic and geographic reporting may be provided without sharing personal information to labs or physicians. The server system may comprise at least two data servers. Data relating to the patient, such as personal information, may be stored separately from the analytical results. For example, the analytical result may be stored in a first data server and the data relating to the patient may be stored on a second data server. Thus, result data can be treated as anonymized. This may provide greater security. The data relating to the patient may not reveal the analytical result. The controlling step may comprise the patient, via the mobile application, granting or denying access to the analytical result and/or to data relating to the patient to other users of the medical system and/or the patient, via the mobile application, assigning access rights to the analytical result and/or to data relating to the patient to other users of the medical system. The patient may provide access information for one or both of accessing the analytical result and the data relating to the patient to the authorization unit via the mobile application. The authorization unit may grant or deny permission based on said access information. The first data server may allow or deny access to the analytical result based on permission from the authorization unit and the second data server may allow or deny access to the data relating to the patient result based on permission from the authorization unit.

In a further aspect, a computer program for performing the mobile application as defined in any one of the embodiments as described herein, while the computer program is being executed on a processor of a mobile device, is disclosed. The computer program may include computer-executable instructions for performing the mobile application, specifically one or more of generating identification data, transmitting the identification data to the authentication unit, providing the sample identity information to the data server, and accessing the data server to access the analytical result. Thus, generally speaking, disclosed and proposed herein is a computer program including computer-executable instructions for performing the mobile application according to the present invention in one or more of the embodiments enclosed herein when the program is executed on a computer or computer network. Specifically, the computer program may be stored on a computer-readable data carrier. The computer specifically may be fully or partially integrated into the mobile device, and the computer programs specifically may be embodied as a software app. Alternatively, however, at least part of the computer may also be located outside the mobile device.

In a further aspect, a computer program comprising program means for fully or partially performing the method as defined in any one of the embodiments as described herein, while the computer program is being executed on a computer or on a computer network of a medical system, is disclosed. The computer program comprises program means for
- receiving identification data by an authentication unit from a mobile application;
- assigning access rights of users of the medical system to access a data server by an authorization unit;
- receiving at least one sample identity information by the data server from the mobile application;
- receiving, upon successful authorization by the authorization unit, at least one analytical result of the biological sample by the data server;
- permitting, upon successful authentication and successful authorization, to access to the analytical result on the data server via the mobile application.

Thus, generally speaking, disclosed and proposed herein is a computer program including computer-executable instructions for performing the method for exchanging health data according to the present invention in one or more of the embodiments enclosed herein when the program is executed on a computer or computer network. Specifically, the computer program may be stored on a computer-readable data carrier. The computer specifically may be fully or partially integrated into one or more entity of the medical system and/or the mobile device, and the computer programs specifically may be embodied as software. Alternatively, however, at least part of the computer may also be located outside the medical system and/or the mobile device.

Further disclosed and proposed herein is a computer program product having program code means, in order to perform the method according to the present invention in one or more of the embodiments enclosed herein when the program is executed on a computer or computer network, e.g. one or more of the method steps mentioned above. Specifically, the program code means may be stored on a computer-readable data carrier.

Further disclosed and proposed herein is a data carrier having a data structure stored thereon, which, after loading into a computer or computer network, such as into a working memory or main memory of the computer or computer network, may execute the method according to one or more of the embodiments disclosed herein, specifically one or more of the method steps mentioned above.

Further disclosed and proposed herein is a computer program product with program code means stored on a machine-readable carrier, in order to perform the method according to one or more of the embodiments disclosed herein, when the program is executed on a computer or computer network, specifically one or more of the method steps mentioned above. As used herein, a computer program product refers to the program as a tradable product. The product may generally exist in an arbitrary format, such as in a paper format, or on a computer-readable data carrier. Specifically, the computer program product may be distributed over a data network.

Finally, disclosed and proposed herein is a modulated data signal which contains instructions readable by a computer system or computer network, for performing the method according to one or more of the embodiments disclosed herein, specifically one or more of the method steps mentioned above.

In a further aspect of the present invention, a mobile device is disclosed. The mobile device comprises at least one processor being programmed for fully or partially executing the method for exchanging health data of a patient in a medical system according to any one of the embodiments described above or described in further detail below referring to a method:
- generating identification data of the patient by a mobile application of the mobile device and transmitting the identification data to an authentication unit by the mobile application;
- associating a biological sample of the patient with the patient, wherein at least one sample identity information is provided to a data server by the mobile application;
- accessing the data server and accessing to the analytical result via the mobile application.
For possible definitions or embodiments, reference may be made to the description of the method as given above.

Specifically, the mobile device may comprise at least one display device configured for displaying at least one item of health information to the patient, the at least one item of health information being based on the analytical result.

The mobile device may comprise at least one tag reader, specifically one or more of a camera or an NFC reader. The tag reader may be configured for identifying the biological sample with the mobile device.

The identification data may be obtained using an OpenID Connect flow and/or may be a phone number. The identification data may be confirmed by code sent by Short Message Service (SMS).

In a further aspect of the present invention, a server system for exchange of health data of a patient in a medical system is disclosed. The medical system is accessible by one or more users including at least the patient. The server system comprises:
- at least one authentication unit configured for receiving identification data generated by a mobile application of a mobile device of the patient and for authentication of the patient;
- at least one data server configured for storing health data;
- at least one authorization unit configured for assigning access rights of the users to access the data server.

Upon successful authorization of a patient by the authorization unit, the data server is configured for receiving at least one sample identity information of a biological sample of the patient. Upon successful authorization by the authorization unit, the data server is configured for receiving at least one analytical result of the biological sample. Upon successful authorization of the patient by the authorization unit, the data server permits the patient to access to the analytical result via the mobile application.

For possible definitions of terms and possible embodiments, reference may be made to the description given above.

Specifically, the authentication unit is configured for providing trusted identity information to the data server and the authorization unit.

The authorization unit may be configured for enabling the patient to grant or deny access to the analytical result to other users of the medical system and/or to assign access rights to the analytical result to other users of the medical system. The authorization unit may be configured to receive access information from the patient. The authorization unit may be configured to grant or deny permission based on said access information. The data server may be configured to allow or to deny access to the analytical result based on permission from the authorization unit. The authorization unit may be configured to determine access rights of the patient and enables the patient to provide the sample identity information to the data server.

The analytical result of the sample may be generated by the at least one analyzer. The authorization unit may be configured to authorize the analyzer to access the data server. The data server may be configured to receive the analytical result from the analyzer. The authentication unit may be configured to receive authentication information from the analyzer and to authenticate the analyzer. The authorization unit, upon successful authentication of the analyzer by the authentication unit, may be configured to determine access rights of the analyzer and to enable the analyzer to provide the analytical result to the data server. The authorization unit may be configured to receive analyzer access information provided by the patient. The authorization unit may be configured to grant or to deny permission to provide the analytical result to the data server based on said analyzer access information. The data server may be configured to allow or to deny providing the analytical result based on permission from the authorization unit.

In a further aspect of the present invention, a medical system for exchange of health data is disclosed. The medical system comprises:
- at least one server system according to any one of the embodiments described above or described in further detail below referring to a server system;
- at least one mobile device according to any one of the embodiments described above or described in further detail below referring to a mobile device.

Again, for possible definitions of terms and possible embodiments, reference may be made to the description given above.

Summarizing and without excluding further possible embodiments, the following embodiments may be envisaged:
Embodiment 1: Method for exchanging health data of a patient in a medical system, the medical system being accessible by one or more users including at least the patient, the method comprising the following steps:
   - at least one authentication step, wherein identification data is generated by a mobile application of a mobile device of the patient, wherein the identification data is transmitted to an authentication unit by the mobile application;
   - at least one authorization step, wherein access rights of the users of the medical system to access a data server are assigned by an authorization unit;
   - at least one association step, wherein, upon successful authentication of the patient and successful authorization of the patient, a biological sample of the patient is associated with the patient, wherein at least one sample identity information is provided to the data server by the mobile application;
   - at least one health data receiving step, wherein, upon successful authorization by the authorization unit, at least one analytical result of the biological sample is received by the data server;
   - at least one health data access step, wherein, upon successful authentication of the patient and successful authorization of the patient, the data server permits the patient to access to the analytical result via the mobile application.
Embodiment 2: The method according to embodiment 1, wherein the method further comprises identifying the biological sample with the mobile device by using a tag reader of the mobile device, specifically one or more of a camera or an NFC reader.
Embodiment 3: The method according to embodiment 2, wherein the mobile device identifies the biological sample by using a tag selected from the group consisting of a barcode and an RFID.
Embodiment 4: The method according to any one of embodiments 1 to 3, wherein the identification data is a phone number, wherein the identification data is confirmed by code sent by Short Message Service (SMS).
Embodiment 5: The method according to any one of embodiment 1 to 4, wherein the method further comprises:
   - at least one controlling step, comprising the patient, via the mobile application, granting or denying access to the analytical result to other users of the medical system and/or the patient, via the mobile application, assigning access rights to the analytical result to other users of the medical system.
Embodiment 6: The method according to embodiment 5, wherein the patient provides access information to the authorization unit via the mobile application, wherein the authorization unit grants or denies permission based on said access information, wherein the data server allows or denies access to the analytical result based on permission from the authorization unit.
Embodiment 7: The method according to embodiment 6, wherein one or both of the health data access step and the controlling step are based on OAuth 2.0 standards and User Managed Access (UMA) protocol.
Embodiment 8: The method according to any one of embodiments 1 to 7, wherein in the authentication step the authentication unit provides trusted identity information to the data server and the authorization unit.
Embodiment 9: The method according to any one of embodiments 1 to 8, wherein the authentication step is based on OpenID Connect standards.
Embodiment 10: The method according to any one of embodiments 1 to 9, wherein the authorization unit, upon successful authentication of the patient, determines access rights of the patient and enables the patient to provide the sample identity information to the data server via the mobile application.
Embodiment 11: The method according to embodiment 10, wherein the association step is based on OAuth 2.0 standards.
Embodiment 12: The method according to any one of embodiments 1 to 12, wherein the method further comprises:
   - at least one analysis step, wherein the analytical result of the biological sample is generated by an analyzer, wherein, if the authorization unit successfully authorizes the analyzer to access the data server, the analyzer provides the analytical result to the data server.
Embodiment 13: The method according to embodiment 12, wherein the method further comprises the analyzer providing authentication information to the authentication unit, wherein the authorization unit, upon successful authentication of the analyzer by the authentication unit, determines access rights of the analyzer and enables the analyzer to provide the analytical result to the data server.
Embodiment 14: The method according to any one of embodiments 12 and 13, wherein the patient provides analyzer access information to the authorization unit via the mobile application, wherein the authorization unit grants or denies permission to provide the analytical result to the data server based on said analyzer access information, wherein the data server allows or denies providing the analytical result based on permission from the authorization unit.
Embodiment 15: The method according to embodiment 14, wherein the health data receiving step is based on OAuth 2.0 standards and User Managed Access (UMA) protocol.
Embodiment 16: The method according to any one of embodiments 12 to 15, wherein the analyzer automatically uploads the analytical result.
Embodiment 17: The method according to any one of embodiments 1 to 16, wherein the method further comprises the mobile application displaying at least one item of health information to the patient by using at least one display device, the at least one item of health information being based on the analytical result.
Embodiment 18: A computer program comprising program means for performing the mobile application as defined in any one of embodiments 1 to 17, while the computer program is being executed on a processor of a mobile device.
Embodiment 19: A computer program comprising program means for fully or partially performing the method according to any one of embodiments 1 to 17, while the computer program is being executed on a computer or on a computer network of a medical system, wherein the computer program comprises program means for
   - receiving identification data by an authentication unit from a mobile application;
   - assigning access rights of users of the medical system to access a data server by an authorization unit;
   - receiving at least one sample identity information by the data server from the mobile application;
   - receiving, upon successful authorization by the authorization unit, at least one analytical result of the biological sample by the data server;
   - permitting, upon successful authentication and successful authorization, to access to the analytical result on the data server via the mobile application.
Embodiment 20: A mobile device, wherein the mobile device comprises at least one processor being programmed for fully or partially executing the method for exchanging health data of a patient in a medical system according to any one of embodiments 1 to 17:
   - generating identification data of the patient by a mobile application of the mobile device and transmitting the identification data to an authentication unit by the mobile application;
   - associating a biological sample of the patient with the patient, wherein at least one sample identity information is provided to a data server by the mobile application;
   - accessing the data server and accessing to the analytical result via the mobile application.
Embodiment 21: The mobile device according to the embodiment 20, wherein the mobile device comprises at least one display device configured for displaying at least one item of health information to the patient, the at least one item of health information being based on the analytical result.
Embodiment 22: The mobile device according to any one of the embodiments 20 to 21, wherein the mobile device comprises at least one tag reader, specifically one or more of a camera or an NFC reader, wherein the tag reader is configured for identifying the biological sample with the mobile device.
Embodiment 23: The mobile device according to any one of the embodiments 20 to 22, wherein the identification data is obtained using an OpenID Connect flow and/or is a phone number, wherein the identification data is confirmed by code sent by Short Message Service (SMS).
Embodiment 24: A server system for exchange of health data of a patient in a medical system, the medical system being accessible by one or more users including at least the patient, the server system comprising:
   - at least one authentication unit configured for receiving identification data generated by a mobile application of a mobile device of the patient and for authentication of the patient;
   - at least one data server configured for storing health data;
   - at least one authorization unit configured for assigning access rights of the users to access the data server,
   wherein, upon successful authorization of a patient by the authorization unit, the data server is configured for receiving at least one sample identity information of a biological sample of the patient, wherein, upon successful authorization by the authorization unit, the data server is configured for receiving at least one analytical result of the biological sample, wherein, upon successful authorization of the patient by the authorization unit, the data server permits the patient to access to the analytical result via the mobile application.
Embodiment 25: The server system according to embodiment 24, wherein the authorization unit is configured for enabling the patient to grant or deny access to the analytical result to other users of the medical system and/or to assign access rights to the analytical result to other users of the medical system.
Embodiment 26: The server system according to embodiment 25, wherein the authorization unit is configured to receive access information from the patient, wherein the authorization unit is configured to grant or deny permission based on said access information, wherein the data server is configured to allow or to deny access to the analytical result based on permission from the authorization unit.
Embodiment 27: The server system according to any one of embodiments 24 to 26, wherein the authentication unit is configured for providing trusted identity information to the data server and the authorization unit.
Embodiment 28: The server system according to any one of embodiments 24 to 27, wherein the authorization unit is configured to determine access rights of the patient and enables the patient to provide the sample identity information to the data server.
Embodiment 29: The server system according to any one of embodiments 24 to 28, wherein the analytical result of the sample is generated by an analyzer, wherein, the authorization unit is configured to authorize the analyzer to access the data server, wherein the data server is configured to receive the analytical result from the analyzer.
Embodiment 30: The server system according to embodiment 29, wherein the authentication unit is configured to receive authentication information from the analyzer and to authenticate the analyzer, wherein the authorization unit, upon successful authentication of the analyzer by the authentication unit, is configured to determine access rights of the analyzer and to enable the analyzer to provide the analytical result to the data server.
Embodiment 31: The server system according to any one of embodiments 29 and 30, wherein the authorization unit is configured to receive analyzer access information provided by the patient, wherein the authorization unit is configured to grant or to deny permission to provide the analytical result to the data server based on said analyzer access information, wherein the data server is configured to allow or to deny providing the analytical result based on permission from the authorization unit.
Embodiment 32: A medical system for exchange of health data, the medical system comprising:
   - at least one server system according to any one of embodiments 24 to 31;
   - at least one mobile device according to any one of embodiments 20 to 23.

### SHORT DESCRIPTION OF THE FIGURES

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1: shows a flow chart of a method for exchanging health data of a patient in a medical system;
- Figure 2: shows a schematic illustration of an embodiment of a medical system according to the present invention; and
- Figures 3A and 3B: show exemplary scenarios in a mobile application according to the present invention.

### DETAILED DESCRIPTION OF THE EMBODIMENTS

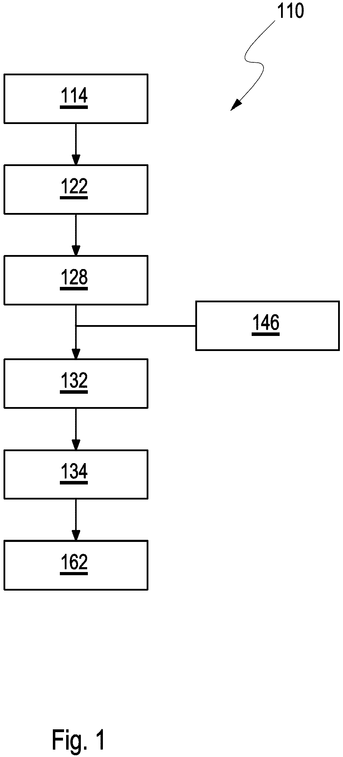

In Figure 1 shows a flow chart of a method 110 for exchanging health data of a patient in medical system 112. The medical system 112 is accessible by one or more users including at least the patient. The method 110 comprises the following steps:
- at least one authentication step 114, wherein identification data is generated by a mobile application 116 of a mobile device 118 of the patient, wherein the identification data is transmitted to an authentication unit 120 by the mobile application 116;
- at least one authorization step 122, wherein access rights of the users of the medical system 112 to access a data server 124 are assigned by an authorization unit 126;
- at least one association step 128, wherein, upon successful authentication of the patient and successful authorization of the patient, a biological sample 130 of the patient is associated with the patient, wherein at least one sample identity information is provided to the data server 124 by the mobile application 116;
- at least one health data receiving step 132, wherein, upon successful authorization by the authorization unit 126, at least one analytical result of the biological sample 130 is received by the data server 132;
- at least one health data access step 134, wherein, upon successful authentication of the patient and successful authorization of the patient, the data server 132 permits the patient to access to the analytical result via the mobile application 116.

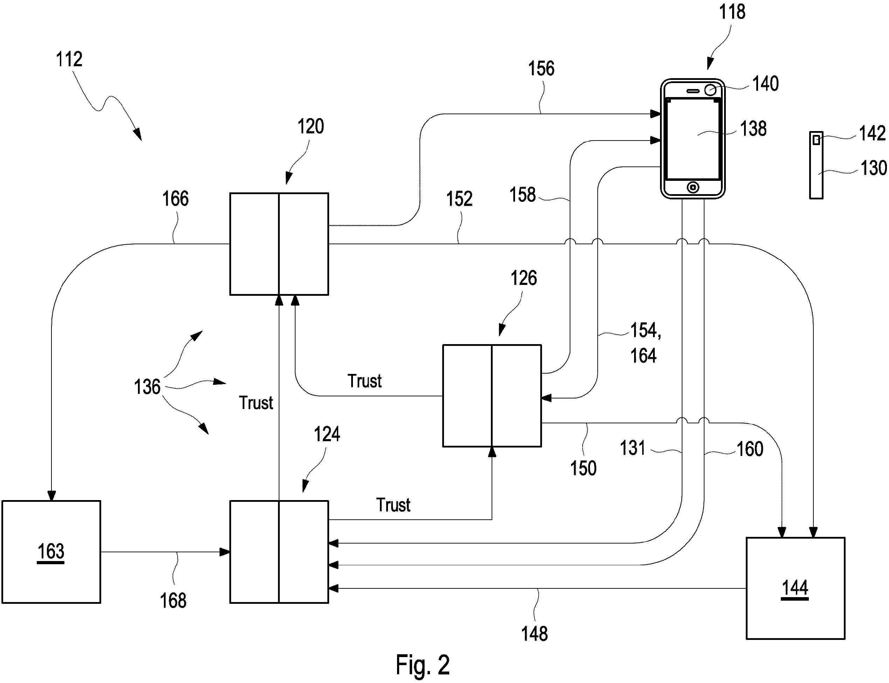

Figure 2 shows a schematic illustration of an embodiment of the medical system 112 which can be used in the method 110 as depicted in Figure 1. The medical system 112 comprises at least one server system 136 and the mobile device 118. The server system 136 comprises the at least one authentication unit 120 configured for receiving identification data generated by the mobile application 116 of the mobile device 118 of the patient and for authentication of the patient, the at least one data server 120 configured for storing health data and the at least one authorization unit 126 configured for assigning access rights of the users to access the data server 120. The mobile device 118 comprises at least one processor (not shown here) being programmed for fully or partially executing the method 110 for exchanging health data of a patient in a medical system 112.

The authentication unit 120 may be a server configured to provide trusted identity information. The authentication unit 120 may act as identity provider. The authentication unit 120, the authorization unit 126 and the data server 124 may form a federation or distributed network, wherein the authorization unit 126 and the data server 124 trust the identity provider to define identities, i.e. to define who is who (denoted with "Trust" in Figure 2). In the authentication step 114, the authentication unit 120 may provide trusted identity information to the data server 124 and the authorization unit 126. For example, the trusted identity information may be and/or may comprise at least one identity token.

The mobile device 118 may comprise at least one data transmission module adapted to transmit the identification data to the authentication unit 120. The identification data may be transmitted from mobile device 118 to the authentication unit 120 by use of a network such as the Internet, a local area network (LAN), a wide area network (WAN), or other type of network. For example, the transmission may use at least one communication protocol such as the transmission control protocol/Internet protocol (TCP/IP).

The authentication step 114 may be based on OpenID Connect standards. The identification data may be obtained using an OpenID Connect flow and/or is a phone number. For example, mobile application 116 may request the patient to enter his or her phone number. The mobile application 116 may be configured to transmit the identification to the authentication unit 120 upon approval and/or consent by the patient, wherein, for example, the approval may be given by pressing at least one button of the mobile device 118 or on a display device 138 of the mobile device 118. The identification data may be confirmed by code sent by Short Message Service (SMS). For example, the authentication unit 120 may be configured to transmit to the mobile device 118 the at least one confirmation code. The confirmation code may be an arbitrary a piece of data such as a sequence of numbers and/or other characters configured for confirmation of the identity of the user. The mobile device 118 may be configured to receive and/or display the confirmation code. The mobile application 116 may be configured to allow the user entering the confirmation code and to proceed with at least one further step of the method 110 such as with the association step 128 or the health data access step 134.

The authorization unit 126 may be configured for access control. The authorization unit 126 may determine which user can do what such as which user is allowed to access data and/or reading data and/or input data. The authorization unit 126 may be configured to control access rights. Specifically, the authorization unit 126 may determine, specifically decide, which data the user should be able to access and/or to which extend user should be able to access the data such as for reading, writing and/or for further actions. The authorization unit 126 may act as an authorization provider. The authorization step 122 may be based on an OAuth standard such an OAuth 2.0 standard. Use of an OAuth standard may allow applications to take actions on the data server 124 on behalf of the respective user, such as may allow the mobile application 116 of the mobile device 118 to take actions on the data server on behalf of the patient.

The data server 124 may comprise at least one data storage device such as at least one memory for storing health data. The data server 124 generally may comprise the at least one data storage device such as at least one volatile and/or at least one non-volatile data storage element. As an example, the data storage device, also referred to as a memory device or a memory element, may comprise one or more storage chips and/or other types of memory devices, wherein both volatile and non-volatile memory devices may be employed. The data server 124 may comprise at least one database comprising the stored health data.

The biological sample 130 may be one or more of a bodily fluid such as blood, urine, saliva, or other bodily fluids, a tissue sample, and other bodily samples such as hair specimen. The biological sample 130 may be a drawn sample of the patient's body. The association step 128 may comprise linking, specifically uniquely linking, the patient with the biological sample 130. In the association step 128, the patient may register the biological sample 130 through the mobile application 116. The authorization unit 126, upon successful authentication of the patient, may determine access rights of the patient and enables the patient to provide the sample identity information to the data server 124 via the mobile application 116 (denoted with arrow 131). The at least one sample identity information is provided to the data server 124 by the mobile application 116. The association step 128 may be based on OAuth 2.0 standards.

The method 110 may further comprise identifying the biological sample 130 with the mobile device 118 by using a tag reader 140 of the mobile device 118, specifically one or more of a camera or a near-field communication (NFC) reader. The mobile device 118 may identify the biological sample 130 by using a tag 142 selected from the group consisting of a barcode and a rapid frequency identification device (RFID). As an example, identifying the biological sample 130 may include reading a number attached or assigned to the biological sample 130 which uniquely identifies the biological sample 130 or characterizes the biological sample 130. Thus, the biological sample 130 may have at least one unique identifier, designated as tag 142, wherein the process of identifying the biological sample 130 implies reading the unique identifier. The tag 142 specifically may be an identifier which is contactless readable, such as an optical identifier and/or a contactless electronic identifier. As an example, the tag 142 may comprise a one-dimensional, a two-dimensional or even a three-dimensional barcode. Additionally or alternatively, the at least one tag 142 may comprise at least one rapid frequency identification device (RFID). Other examples are possible. The tag reader 140 specifically may be configured for reading the tag 142. For reading the tag 142, the tag 142 may be brought into a field of view of the tag reader 140. As an example, the tag reader 140 may be configured as a camera, specifically a barcode reader, and/or as an NFC reader, specifically an RFID tag reader. The tag reader 140 specifically may comprise at least one optical reader. As an example, the tag reader 140 may comprise at least one barcode reader. The optical reader, as an example, may be a camera, specifically a scanner or may comprise a scanner, such as a two-dimensional scanner or a line-scanner. As an example, the tag reader may be an NFC reader. The NFC reader may comprise at least one communication component adapted to perform the near-field communication. As an example, the NFC reader may comprise at least one antenna. As an example, the NFC reader device may comprise at least one RFID antenna, such as at least one RFID coil. The NFC reader may further comprise additional components, such as one or more communication ICs or the like.

The method 110 may further comprise transmitting the biological sample 130 to an analyzer 144 such as a laboratory for testing and/or analyzing the biological sample 130. The method 110 further may comprise at least one analysis step 146, wherein the analytical result of the biological sample 130 is generated by the analyzer 144. The analyzer 144 may be or may comprise at least one arbitrary device configured for conducting at least one medical analysis and/or at least one medical procedure. The analyzer 144 therefore may generally be an arbitrary device configured for performing at least one diagnostic purpose and/or at least one therapeutic purpose. The analyzer 144 may be configured for performing at least one diagnostic purpose and, specifically may comprise at least one analyte sensor for performing at least one analysis. The analyzer 144 may comprise an assembly of two or more components capable of interacting with each other, such as in order to perform one or more diagnostic and/or therapeutic purposes, such as in order to perform the medical analysis and/or the medical procedure. Specifically, the analyzer 144 may be capable of performing at least one detection of the at least one analyte in the biological sample 130 and/or in order to contribute to the at least one detection of the at least one analyte in the biological sample 130. The analyzer 144 generally may be used for detecting at least one analyte in the biological sample 130 of the patient. The analyzer 144 may be or may comprise at least one of a sensor assembly, a sensor system, a sensor kit or a sensor device.

For example, the analyzing may comprise at least one process of determining a presence and/or a quantity and/or a concentration of the at least one analyte. The analyte may be a component of a sample to be analyzed, e.g. molecules of various sizes, ions, proteins, metabolites and the like. Information gathered on an analyte may be used to evaluate the impact of the administration of drugs on the organism or on particular tissues or to make a diagnosis. Examples of analytes are e.g. glucose, coagulation parameters, endogenic proteins (e.g. proteins released from the heart muscle), metabolites, nucleic acids and so on. The list of possible analysis results returned by the analyzer may comprise, without limitation, concentrations of the analyte in the sample, a digital (yes or no) result indicating the existence of the analyte in the sample (corresponding to a concentration above the detection level), optical parameters, DNA or RNA sequences, data obtained from mass spectrometry of proteins or metabolites and physical or chemical parameters of various types. Thus, the analysis may be or may comprise a qualitative detection, simply determining the presence of the at least one analyte or the absence of the at least one analyte, and/or may be or may comprise a quantitative detection, which determines the quantity and/or the concentration of the at least one analyte. As a result of the analysis, at least one information may be generated which characterizes an outcome of the analysis, such as at least one test result, e.g. a blood value, a blood test result, presence or absence of the analyte, or other health information.

The receiving of the analytical result may comprise storing the analytical result in the data server 124. Once the analytical result is ready, the analytical result can be provided to the patient. Upon successful authorization by the authorization unit 126, the at least one analytical result of the biological sample 130 is received by the data server 124. The analyzer 144 may automatically and/or directly upload the analytical result to the data server 124, denoted with arrow 148. This may remove patient action and, thus, may reduce user errors and making it more convenient for the patient. If the authorization unit 126 successfully authorizes (denoted with arrows 150) the analyzer to access the data server 124, the analyzer 144 may provide the analytical result to the data server 124. The method 110 further may comprise the analyzer 144 providing authentication information to the authentication unit 120. The authorization unit 126, upon successful authentication of the analyzer 144 by the authentication unit (denoted with arrow 152), may determine access rights of the analyzer 144 and may enable the analyzer 144 to provide the analytical result to the data server 124. The patient may provide analyzer access information to the authorization unit 126 via the mobile application 116 (denoted with arrow 154). The authorization unit 126 may grant or deny permission to provide the analytical result to the data server 124 based on said analyzer access information. The data server 124 may allow or deny providing the analytical result based on permission from the authorization unit 126. The health data receiving step 132 may be based on OAuth 2.0 standards and User Managed Access (UMA) protocol.

In the health data access step 134, upon successful authentication of the patient (denoted with arrow 156) and successful authorization of the patient (denoted with arrow 158), the data server 124 permits the patient to access to the analytical result via the mobile application 116 (denoted with arrow 160). The data server 124 may issue a notification to the patient that analytical results were uploaded and/or are available on the data server 124 via the mobile application 116. The method 110 further may comprise the mobile application 116 displaying at least one item of health information to the patient by using the at least one display device 138, the at least one item of health information being based on the analytical result.

The method 110 may further comprise at least one controlling step 162. The controlling step 162 may comprise the patient, via the mobile application 116, granting or denying access to the analytical result to other users 163 of the medical system 112 and/or the patient, via the mobile application 116, assigning access rights to the analytical result to other users 163 of the medical system 112. For example, once the analytical result is available on the data server 124, the mobile application 116 can access the data server 124 to download the analytical result. The mobile application 116 may allow the patient to grant or deny access to the analytical result to other users 163 such as health care professionals. The patient may provide access information to the authorization unit 126 via the mobile application 116 (denoted with arrow 164). The authorization unit 126 may grant or deny permission based on said access information, specifically after authentication of the other user (denoted with arrow 166). The data server 124 may allow or deny access to the analytical result based on permission from the authorization unit 126 (denoted with arrow 168). One or both of the health data access step 134 and the controlling step 162 may be based on OAuth 2.0 standards and User Managed Access (UMA) protocol. The UMA protocol may define a method for the patient to introduce the health data to the authorization unit 126. The UMA protocol may define at least one policy for controlling access to the health data on the data server 124. The UMA protocol may request users of the medical system, e.g. the patient and other users 163 of the medical system 112, to provide claims to fulfill the defined policies for accessing the data server 124, specifically the health data. The use of the UMA protocol may allow ensuring and increasing baseline security. In addition, the use of UMA protocol may enhance interoperability between entities of the medical system 112.

The method 110 for exchange of health data may use the server system 136 comprising authentication unit 120, authorization unit 126 and data server 124 which may form a federation or distributed network. For user access the method 110 may use the HEART standards, specifically OpenID Connect to identify users, OAuth 2.0 to delegate access to trusted applications and UMA to control who can access the data. This may allow patient managed access. Patients can manage their consent online. Granting and retracting consent may be equally easy for patients. The use of the HEART standard may allow relying on existing identity and delegation standards, which are compliant with General Data Protection Regulation (GDPR). All parties agree on identities, so systems can exchange data with authorized people.

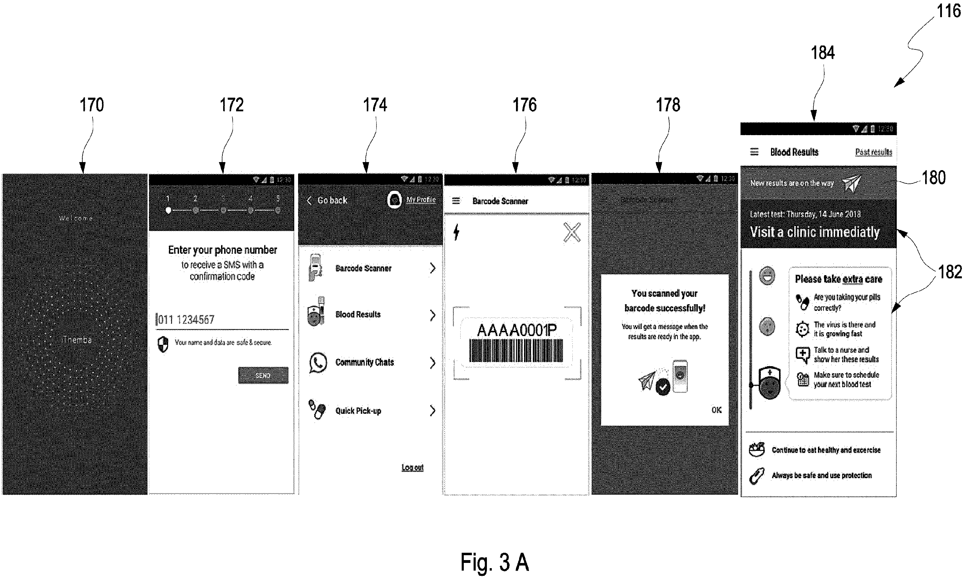

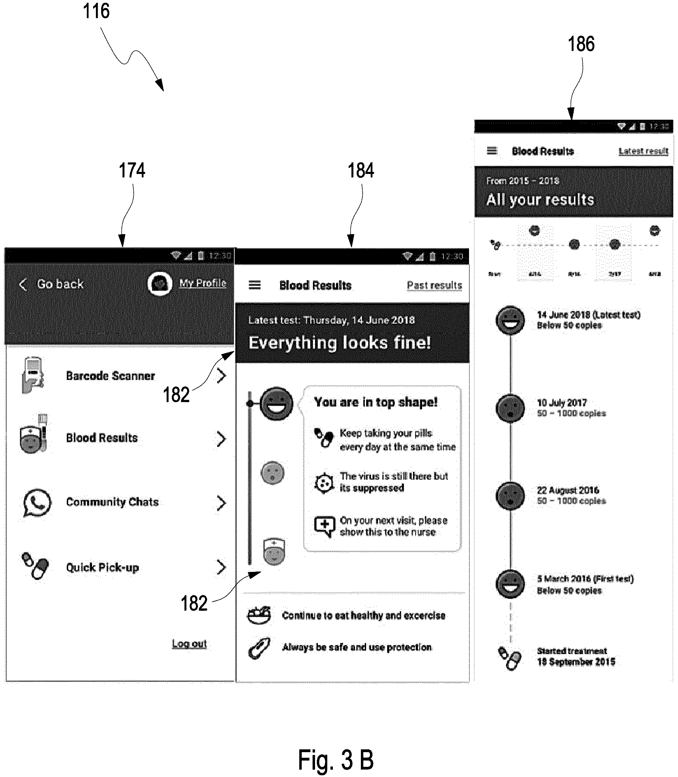

Figures 3A and 3B show exemplary scenarios in the mobile application 116. The display device 138 may display the depicted views, in particular screenshots, in the shown order such as a timely order from left to right. For example, in the scenario shown in Figure 3A, after executing the mobile application 116 the mobile application 116 may display an indication that the mobile application 116 can be used such as a "Welcome" view shown in the left view 170 of Figure 3A.

Subsequent four views of Figure 3A show an example of the authentication step 114 and the association step 128. Thus, the mobile application 116 may request the patient to enter his or her phone number (view 172). Upon entering the phone number, the mobile application 116 may generate identification data and may transmit the identification data to the authentication unit 120. The mobile application 116 may be configured to transmit the identification to the authentication unit 120 upon approval and/or consent by the patient, wherein, for example, the approval may be given by pressing at least one button of the mobile device 118 or on the display device 138. The identification data may be confirmed by code sent by Short Message Service (SMS). For example, the authentication unit 120 may be configured to transmit to the mobile device 118 the at least one confirmation code. The confirmation code may be an arbitrary a piece of data such as a sequence of numbers and/or other characters configured for confirmation of the identity of the user. The mobile device 118 may be configured to receive and/or display the confirmation code. The mobile application 116 may be configured to allow the patient entering the confirmation code and to proceed with at least one further step of the method 110.

Next, the mobile application 116 may display at least one menu overview 174. The menu overview 174 may display possible menu options the patient can select such as registration of the biological sample 130 denoted as "Barcode Scanner", access to health data denoted "Blood Results", communication with third parties, denoted as "Community Chat" in the Figure, and access to event history, denoted as "Quick Pick-Up" in the Figure.

For example, the patient may select the option "Barcode Scanner". This option may allow the patient to register the biological sample 130. The mobile application may request the patient to identify the biological sample 130 with the mobile device 118 by using the tag reader 140. The mobile application 116 may display the recorded sample identity information, see view 176, specifically for review for the patient. The mobile application 116 may display a confirmation to the patient that the barcode was scanned successfully, see view 178.

For example, the patient may select the option "Blood Results", specifically after notification 180 by the mobile application 116 that new analytical results are available on the data server 124. The mobile application 116 may be configured for accessing the data server 124 and to access the health data stored therein. The mobile application 116 may display at least one item of health information 182 to the patient by using the at least one display device 138, see view 184, the at least one item of health information 182 being based on the analytical result. The item of health information 182 may comprise at least one notification and/or at least one request. For example, in case of alarming or critical analytical result, the mobile application 116 may issue a request to visit a clinic immediately. The item of health information 182 may comprise requests and/or calls to action such as at least one reminder. For example, the item of health information 182 may comprise at least one reminder to take a medicine and/or at least one reminder or at least one request to contact to a medical professional and/or at least one reminder or at least one request to schedule a further blood test. Additionally or alternatively, the item of health information 182 may comprise color information depending on the analytical result. The color information may be a color selected from a color gradient, wherein the color gradient may range from green for non-critical results to red for critical results. The displayed color may depend on a level of criticality and/or urgency. For example, in the scenario shown in Figure 3A, wherein the analytical result is alarming or critical the selected color may be "red". Additionally or alternatively, the item of health information 182 may comprise at least one emotion icon and/or a graphical representation of at least one emotion icon. The displayed emotion icon and/or graphical representation of the emotion icon may depend on a level of criticality and/or urgency. Using color information and/or emotion icon and/or a graphical representation of at least one emotion icon may allow the patient to immediately recognize health state.

Figure 3B represents another possible scenario in the mobile application, wherein the three first screenshots 170, 172, 174 are the same as in the previous scenario and wherein only screenshot 174 is shown in Figure 3B. For example, the patient may select in the menu overview 174 the option "Blood Results". The mobile application 116 may be configured for accessing the data server 124 and to access the health data stored therein.

The mobile application 116 may display the item of health information 182 to the patient by using the at least one display device 138, see view 184, the at least one item of health information 182 being based on the analytical result. In the scenario of Figure 3B, the item of health information 182 may be information that the analytical result is non-critical such as a notification "Everything looks fine!". The mobile application 116 may display further information e.g. further requests or calls to action such as at least one reminder. For example, the further or additional information may comprise at least one reminder to take a medicine and/or at least one reminder or at least one request to contact to a medical professional and/or at least one reminder or at least one request to schedule a further blood test. As outlined above, the item of health information 182 may comprise color information depending on the analytical result. For example, in the scenario shown in Figure 3B, wherein the analytical result is non-critical the selected color may be "green". Additionally or alternatively, the item of health information 182 may comprise the at least one emotion icon and/or the graphical representation of the at least one emotion icon. The displayed emotion icon and/or graphical representation of the emotion icon may depend on a level of criticality and/or urgency.

The mobile application 116 further may display history of analytical test results, see screenshot 186. This may allow the patient to determine development and/or overview of his or her health status. The history of analytical test results may comprise information of date of the analytical test result. The history of analytical test results may comprise at least one graphical information about a development of the analytical result and/or the health status such as a diagram showing timely development of the analytical result and/or the health status. For example, the graphical information may comprise timely development of the health status, wherein the health status may be represented by the respective emotion icon and/or the respective graphical representation of the emotion icon. This may allow the patient to immediately recognizing development of health state without need of complicated diagrams.

### List of reference numbers

- 110: method
- 112: medical system
- 114: authentication step
- 116: mobile application
- 118: mobile device
- 120: authentication unit
- 122: authorization step
- 124: data server
- 126: authorization unit
- 128: association step
- 130: biological sample
- 131: arrow
- 132: health data receiving step
- 134: health data access step
- 136: server system
- 138: display device
- 140: tag reader
- 142: tag
- 144: analyzer
- 146: analysis step
- 148: upload
- 150: authorization of analyzer
- 152: authentication of analyzer
- 154: providing analyzer access information
- 156: authentication of patent
- 158: authorization of patent
- 160: access to data server
- 162: controlling step
- 163: other users
- 164: providing access information
- 166: authentication of other user
- 168: access to data server
- 170: welcome view
- 172: view
- 174: menu overview
- 176: view
- 178: view
- 180: notification
- 182: item of health information
- 184: view
- 186: history

## Claims

1. Method (110) for exchanging health data of a patient in a medical system (112), the medical system (112) being accessible by one or more users including at least the patient, the method (110) comprising the following steps:
- at least one authentication step (114), wherein identification data is generated by a mobile application (116) of a mobile device (118) of the patient, wherein the identification data is transmitted to an authentication unit (120) by the mobile application (116);
- at least one authorization step (122), wherein access rights of the users of the medical system (112) to access a data server (124) are assigned by an authorization unit (126);
- at least one association step (128), wherein, upon successful authentication of the patient and successful authorization of the patient, a biological sample (130) of the patient is associated with the patient, wherein at least one sample identity information is provided to the data server (124) by the mobile application (116);
- at least one health data receiving step (132), wherein, upon successful authorization by the authorization unit (126), at least one analytical result of the biological sample (130) is received by the data server (124);
- at least one health data access step (134), wherein, upon successful authentication of the patient and successful authorization of the patient, the data server (124) permits the patient to access to the analytical result via the mobile application (116).

2. The method (110) according to claim 1, wherein the method (110) further comprises identifying the biological sample (110) with the mobile device (118) by using a tag reader (140) of the mobile device (118.

3. The method (110) according claim 2, wherein the mobile device (118) identifies the biological sample (130) by using a tag (142) selected from the group consisting of a barcode and an RFID, wherein the identification data is a phone number, wherein the identification data is confirmed by code sent by Short Message Service (SMS).

4. The method (110) according to any one of claims 1 to 3, wherein the method (110) further comprises:
- at least one controlling step (162), comprising the patient, via the mobile application (116), granting or denying access to the analytical result to other users of the medical system (112) and/or the patient, via the mobile application (116), assigning access rights to the analytical result to other users of the medical system (112).

5. The method (110) according to claim 4, wherein the patient provides access information to the authorization unit (126) via the mobile application (116), wherein the authorization unit (126) grants or denies permission based on said access information, wherein the data server (124) allows or denies access to the analytical result based on permission from the authorization unit (126).

6. The method (110) according to any one of claims 1 to 5, wherein the authorization unit (126), upon successful authentication of the patient, determines access rights of the patient and enables the patient to provide the sample identity information to the data server (124) via the mobile application (116).

7. The method (110) according to any one of claims 1 to 6, wherein the method (110) further comprises:
- at least one analysis step (146), wherein the analytical result of the biological sample (130) is generated by an analyzer (144), wherein, if the authorization unit (126) successfully authorizes the analyzer (144) to access the data server (124), the analyzer (144) provides the analytical result to the data server (124).

8. The method (110) according to the claim 7, wherein the analyzer (144) automatically uploads the analytical result.

9. A computer program comprising program means for performing the mobile application (116) as defined in any one of claims 1 to 8, while the computer program is being executed on a processor of a mobile device (118).

10. A computer program comprising program means for fully or partially performing the method (110) according to any one of claims 1 to 8, while the computer program is being executed on a computer or on a computer network of a medical system (112), wherein the computer program comprises program means for
- receiving identification data by an authentication unit (120) from a mobile application (116);
- assigning access rights of users of the medical system (112) to access a data server (124) by an authorization unit (126);
- receiving at least one sample identity information by the data server (124) from the mobile application (110);
- receiving, upon successful authorization by the authorization unit (110), at least one analytical result of the biological sample (130) by the data server (124);
- permitting, upon successful authentication and successful authorization, to access to the analytical result on the data server (124) via the mobile application (116).

11. A mobile device (118), wherein the mobile device (118) comprises at least one processor being programmed for fully or partially executing the method (110) for exchanging health data of a patient in a medical system (112) according to any one of claims 1 to 8:
- generating identification data of the patient by a mobile application (116) of the mobile device and transmitting the identification data to an authentication unit (120) by the mobile application (116);
- associating a biological sample (130) of the patient with the patient, wherein at least one sample identity information is provided to a data server (124) by the mobile application (116);
- accessing the data server (124) and accessing to the analytical result via the mobile application (116).

12. The mobile device (118) according to claim 11, wherein the mobile device (118) comprises at least one display device (138) configured for displaying at least one item of health information to the patient, the at least one item of health information being based on the analytical result.

13. The mobile device (118) according to any one of claims 11 and 12, wherein the mobile device (138) comprises at least one tag reader (140), wherein the tag reader (140) is configured for identifying the biological sample (130) with the mobile device (140).

14. The mobile device (118) according to any one of claims 11 to 13, wherein the identification data is obtained using an OpenID Connect flow and/or is a phone number, wherein the identification data is confirmed by code sent by Short Message Service (SMS).

15. A server system (136) for exchange of health data of a patient in a medical system (112), the medical system (112) being accessible by one or more users including at least the patient, the server system (112) comprising:
- at least one authentication unit (120) configured for receiving identification data generated by a mobile application (116) of a mobile device (118) of the patient and for authentication of the patient;
- at least one data server (124) configured for storing health data;
- at least one authorization unit (126) configured for assigning access rights of the users to access the data server (124),
wherein, upon successful authorization of a patient by the authorization unit (126), the data server (124) is configured for receiving at least one sample identity information of a biological sample (130) of the patient, wherein, upon successful authorization by the authorization unit (126), the data server (124) is configured for receiving at least one analytical result of the biological sample (130), wherein, upon successful authorization of the patient by the authorization unit (126), the data server (124) permits the patient to access to the analytical result via the mobile application (116).

16. The server system (136) according to claim 15, wherein the authorization unit (126) is configured for enabling the patient to grant or deny access to the analytical result to other users of the medical system (112) and/or to assign access rights to the analytical result to other users of the medical system (112), wherein the authorization unit (126) is configured to receive access information from the patient, wherein the authorization unit (126) is configured to grant or deny permission based on said access information, wherein the data server (124) is configured to allow or to deny access to the analytical result based on permission from the authorization unit (126).

17. A medical system (112) for exchange of health data, the medical system (112) comprising:
- at least one server system (136) according to any one of claims 15 and 16;
- at least one mobile device (118) according to any one of the claims 11 to 14.
